(19) Europäisches Patentamt
European Patent Office
Office européen des brevets

(11) EP 0 947 493 B1

(12) **EUROPÄISCHE PATENTSCHRIFT**

(45) Veröffentlichungstag und Bekanntmachung des Hinweises auf die Patenterteilung:
**30.10.2002 Patentblatt 2002/44**

(51) Int Cl.[7]: **C07C 45/62**, C07C 47/21, B01J 8/20, B01J 8/22

(21) Anmeldenummer: **99105126.9**

(22) Anmeldetag: **26.03.1999**

(54) **Verfahren zur selektiven Flüssigphasenhydrierung von alpha,beta-ungesättigten Carbonylverbindungen**

Process for the selective liquid phase hydrogenation of alpha,beta-unsaturated carbonyl compounds

Procédé pour l'hydrogénation sélective en phase liquide de composés carbonyles alpha,bèta-insaturés

(84) Benannte Vertragsstaaten:
**AT BE CH DE DK ES FI FR GB GR IE IT LI LU NL PT SE**
Benannte Erstreckungsstaaten:
**RO SI**

(30) Priorität: **02.04.1998 DE 19814879**

(43) Veröffentlichungstag der Anmeldung:
**06.10.1999 Patentblatt 1999/40**

(73) Patentinhaber: **BASF AKTIENGESELLSCHAFT**
**67056 Ludwigshafen (DE)**

(72) Erfinder:
- **Bröcker, Franz Josef, Dr.**
  **67061 Ludwigshafen (DE)**
- **Kaibel, Gerd, Dr.**
  **68623 Lampertheim (DE)**
- **Aquila, Werner, Dr.**
  **68309 Mannheim (DE)**
- **Fuchs, Hartwig**
  **67063 Ludwigshafen (DE)**
- **Wegner, Günter, Dr.**
  **67354 Römerberg (DE)**
- **Stroezel, Manfred**
  **68549 Ilvesheim (DE)**

(74) Vertreter: **Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(56) Entgegenhaltungen:

| | |
|---|---|
| **EP-A- 0 024 651** | **EP-A- 0 798 039** |
| **DD-A- 226 872** | **DE-A- 2 936 362** |
| **DE-A- 19 530 329** | **DE-A- 19 641 707** |
| **FR-A- 2 247 445** | **US-A- 4 847 016** |

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft die Verwendung eines gepackten Blasensäulenreaktors zur selektiven Flüssigphasenhydrierung von α,β-ungesättigten Carbonylverbindungen der Formel I

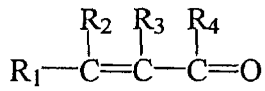 (I)

zu den entsprechenden gesättigten Carbonylverbindungen der Formel II

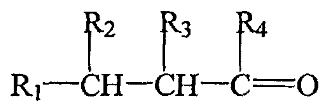 (II)

mit Wasserstoff in Gegenwart eines pulverförmigen Palladiumkatalysators und einer organischen Base. Speziell betrifft die Erfindung die Verwendung zur selektiven Hydrierung von Citral zu Citronellal.

Selektive Hydrierverfahren von α,β-ungesättigten Carbonylverbindungen mit Wasserstoff in der Flüssigphase werden beispielsweise in DE-A-21 14 211 und DE-A-28 39 474 beschrieben. In beiden Verfahren wird diskontinuierlich in Gegenwart eines Palladiumkatalysators und einer Base gearbeitet, wobei nach dem Verfahren der DE-A-28 39 474 mit 15 bis 50 Gew.-% eines tertiären Amins, bezogen auf das Edukt, die Selektivität sowie die Raumzeitausbeute der Hydrierungsreaktion verbessert werden.

Auch mit dieser Verbesserung sind die Reaktionszeiten dennoch unbefriedigend. Sie können zwar durch Verwendung größerer Mengen des Palladiumkatalysators verkürzt werden, dies ist jedoch wegen der hohen Katalysatorkosten unwirtschaftlich, zudem sind größere Katalysatormengen im Hinblick auf die Probleme der Handhabung von Feststoffen nachteilig. Auch ist eine erfolgreiche Regenerierung des desaktivierten Katalysators im allgemeinen nicht möglich.

Aufgabe der Erfindung war es daher, α,β-ungesättigte Carbonylverbindungen der Formel (I) auf wirtschaftlichere Weise zu den entsprechenden α,β-ungesättigten Carbonylverbindungen der Formel (II) zu hydrieren. Dabei sollen die Raumzeitausbeute verbessert und die Investitionskosten reduziert werden.

In einer Ausgestaltung war es Aufgabe der Erfindung, ein wirtschaftlicheres Verfahren zur Hydrierung von Citral zu Citronellal zur Verfügung zu stellen.

Die Lösung besteht in der Verwendung eines gepackten Blasensäulenreaktors, dessen Zuführeinrichtung ein Gasstrahlverdichter ist, mit Produktrückführung und Wasserstoffkreisgas zur selektiven Flüssigphasenhydrierung von α,β-ungesättigten Carbonylverbindungen der allgemeinen Formel I,

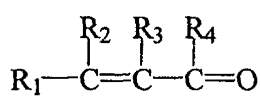 (I)

in der $R_1$ Wasserstoff oder ein organischer Rest, $R_2$, $R_3$ und $R_4$ unabhängig voneinander Wasserstoff oder eine $C_1$- bis $C_4$-Alkylgruppe ist, zu gesättigten Carbonylverbindungen der Formel II

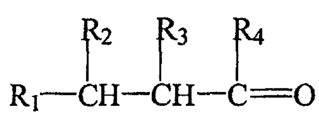 (II)

mit Wasserstoff in Gegenwart eines pulverförmigen Palladiumkatalysators und einer organischen Base.

Ein Reaktor, wie er fiir die erfindungsgemäße Verwendung gefunden wurde, ist aus EP-A 0 798 039 bekannt: Es handelt sich hierbei um eine gepackte Blasensäule, durch die eine Flüssigkeit, die einen suspendierten Feststoffkatalysator enthält, umgepumpt wird, wobei gleichzeitig durch die gepackte Blasensäule ein Wasserstoffkreisgas geführt wird. Überraschend wurde bei der erfindungsgemäßen Verwendung dieses Reaktors zur selektiven Flüssigphasenhydrierung von α, β-ungesättigten Carbonylverbindungen allein die Doppelbindung selektiv hydriert, die Carbonylgruppe dagegen erhalten.

Es wurde gefunden, daß der geschwindigkeitsbestimmende Schritt des Gesamtverfahrens, die Diffusion des gasförmigen Wasserstoffs an die Katalysatorgrenzfläche, dadurch beschleunigt werden kann, daß das Verfahren in einem gepackten Blasensäulenreaktor mit Produktrückführung und Wasserstoffkreisgas durchgeführt wird. Mit fortschreitender Reaktion verarmt die Flüssigkeit in der Nähe der Katalysatorgrenzfläche an Wasserstoff. Dieser wasserstoffarme Film an der Katalysatorgrenzfläche kann nun, durch erfindungsgemäße Verwendung eines gepackten Blasensäulenreaktors mit Produktrückführung und Wasserstoffkreisgas, umgewirbelt werden, so daß ein Austausch mit wasserstoffgesättigter Flüssigkeit von außerhalb ermöglicht wird. Eine entscheidende Rolle spielt dabei die verstärkte Relativbewegung der Katalysatorteilchen gegenüber der Flüssigphase und den Wasserstoffgasblasen, die durch Abbremsungen und kurzzeitiges Festhalten der Katatalysatorteilchen an den Kanalwandungen der Packung bewirkt wird. Durch die verbesserte Hydrodynamik wird der Katalysator besonders gut ausgenutzt.

Die erfindungsgemäße Verwendung ist grundsätzlich auf alle α,β-ungesättigten Carbonylverbindungen der Formel (I) anwendbar, wobei durch die verkürzte Reaktionszeit die Selektivität bezüglich der Hydrierung der Doppelbindung, d.h. der Teilreaktion mit der größeren Geschwindigkeitskonstanten verbessert wird. In einer bevorzugten Ausgestaltung wird das Edukt Citral zu Citronellal umgesetzt.

Der pulverförmige Palladiumkatalysator kann als Vollkatalysator oder Trägerkatalysator eingesetzt werden, wobei als bevorzugte Trägermaterialien Kohlenstoff, Zirkoniumdioxid oder Titandioxid verwendet werden. Besonders vorteilhaft ist es, Katalysatorträger mit einer mittleren Korngröße von 0,1 bis 300 μm, vorzugsweise 0,5 bis 100 μm, einzusetzen. Diese Katalysatorpartikel führen mit ihrer hohen volumenbezogenen Oberfläche zu guten Raumzeitausbeuten, denn sie können beim Durchströmen der Öffnungen und Kanäle der Packungen im Blasensäulenreaktor Relativbewegungen zur Flüssigphase und zu den Wasserstoffgasblasen ausführen.

Die Hydrierung wird in einem gepackten Blasensäulenreaktor durchgeführt. Besonders geeignet sind Pakkungen mit Öffnungen oder Kanälen, deren hydraulischer Durchmesser 0,5 bis 20 mm, vorzugsweise 1 bis 10 mm, besonders bevorzugt 1 bis 3 mm, beträgt. Der hydraulische Durchmesser wird definiert als Quotient des vierfachen Querschnitts der Öffnung und deren Umfang. Die suspendierten Katalysatorteilchen werden in den Öffnungen oder Kanälen der Packung durch Kollisionen mit den Kanalwandungen sowie durch kurzes Festhalten abgebremst. Es wurde festgestellt, daß bei hydraulischen Durchmessern im oben angegebenen Bereich ein Anteil von etwa 15 bis 16 Gew.-% des Katalysators im zeitlichen Mittel auf den Packungswandungen festgehalten wird.

Dieser Effekt kann durch Erhöhung der Oberflächenrauhigkeit der Wandungen noch verbessert werden. Bevorzugt werden Wandmaterialien mit Oberflächenrauhigkeiten im Bereich des 0,1 bis 10fachen, vorzugsweise des 0,5 bis 5fachen der mittleren Korngröße der suspendierten Katalysatorteilchen. Besonders geeignet sind metallische Wandmaterialien, deren Oberfläche einen Mittenrauhwert $R_a$ nach DIN 4768/1 von 0,001 bis 0,01 mm aufweisen.

Als Packungsmaterialien können metallische Werkstoffe, Kunststoffe, Keramik und/oder anorganische Fasern, insbesondere Kohlenstoff oder Asbestersatzstoffe, verwendet werden.

Die Packungen können als Folien, Gestricke oder Gewebe ausgebildet sein, wie sie grundsätzlich, d.h. ihrer geometrischen Form nach, bereits aus der Destillations- oder Extraktionstechnik bekannt sind. Derartige Packungselemente, die den Vorteil eines geringen Druckverlusts bieten, sind zum Beispiel Drahtgewebepackungen der Bauart Montz A3 und Sulzer BX, DX und EX. Für die Zwecke der vorliegenden Erfindung haben die Packungen jedoch grundsätzlich einen wesentlich, regelmäßig um den Faktor 2 bis 10 kleineren hydraulischen Durchmesser als vergleichbare Einbauten im Bereich der Destillations- oder Extraktionstechnik. Drahtgewebepackungen sind besonders vorteilhaft. Statt Gewebepackungen können aber im Rahmen der vorliegenden Erfindung auch Packungen aus anderen gewebten, gewirkten oder gefilzten flüssigkeitsdurchlässigen Materialien verwendet werden. Bei weiteren geeigneten Pakkungen werden ebene Bleche, bevorzugt ohne Perforationen oder andere größere Öffnungen, eingesetzt, beispielsweise entsprechend den Bauarten Montz B1 oder Sulzer Mellapak. Vorteilhaft sind auch Packungen aus Streckmetall, zum Beispiel Packungen des Typs Montz BSH. Auch dabei müssen Öffnungen, wie etwa Perforationen, entsprechend klein gehalten werden. Entscheidend für die Eignung einer Packung im Rahmen der vorliegenden Erfindung ist nicht deren Geometrie, sondern es sind die für die Stromführung entstehenden Öffnungsgrößen bzw. Kanalbreiten in der Packung.

Bevorzugt wird die Flüssigphase mit einer Leerrohrgeschwindigkeit von 100 bis 500 $m^3/m^2.h$, vorzugsweise 150 bis 300 $m^3/m^2.h$ durch den gepackten Blasensäulenreaktor umgepumpt.

Das Wasserstoffkreisgas wird der Flüssigphase mit suspendiertem pulverförmigen Katalysator bevorzugt mit einer Leerrohrgeschwindigkeit von 0,5 bis 15 cm/s, vorzugsweise 2,5 bis 10 cm/s zugeführt. Die Zuführung des Wasserstoffkreisgases erfolgt bevorzugt über einen Gasstrahlverdichter, der eine intensive Vermischung mit der Flüssigphase und dem darin suspendierten Katalysator bewirkt.

Die Hydrierung wird bevorzugt bei Wasserstoffpartialdrücken von 1 bis 200 bar, vorzugsweise 1 bis 100 bar, besonders bevorzugt 1 bis 10 bar, durchgeführt.

Bevorzugte Reaktionstemperaturen sind 25 bis 150°C, vorzugsweise 50 bis 100°C.

Das Verfahren kann sowohl diskontinuierlich als auch kontinuierlich betrieben werden. Besonders vorteilhaft ist die kontinuierliche Fahrweise. Dabei kann der verbrauchte Katalysator nach dem besonders günstigen Verfahren der Querstromfiltration abgetrennt werden.

Die Flüssigphase mit suspendierten Katalysator sowie der Wasserstoff werden bevorzugt im Gleichstrom umgepumpt. Besonders vorteilhaft ist, die Edukte von unten einem vertikal stehend angeordneten Blasensäulenreaktor zuzuführen.

Die Erfindung wird im folgenden anhand einer Figur und eines Ausführungsbeispiels näher erläutert. Es zeigen im einzelnen:

Figur 1 die schematische Darstellung einer Anlage fiir ein diskontinuierliches Verfahren gemäß der Erfindung und

Figur 2 die schematische Darstellung einer Anlage für die besonders bevorzugte kontinuierliche Durchführung des Verfahrens nach der Erfindung.

Figur 1 zeigt beispielhaft die schematische Darstellung einer Anlage mit einem diskontinuierlich betriebenen Blasensäulenreaktor 1, der mit einer Packung 2 bestückt ist, deren Geometrie mit jener der Destillationspackung Montz-Pak-Typ A3-1200 vergleichbar ist.

Zur Durchführung der Hydrierung wird zunächst der Vorratsbehälter 7 über die Befülleitung 3 mit Edukt, Amin und Suspensionskatalysator gefüllt. Mittels der Kreislaufpumpe 12 wird das Reaktionsgemisch über den Vorheizer 13 und den Gasstrahlverdichter 5 in den Reaktor gepumpt und von dort über die Kreislaufleitung 6 in den Vorratsbehälter 7 zurück. Hier wird der nicht umgesetzte Wasserstoff abgetrennt und über die Kreisgasleitung 9 zur Mischdüse 5 und damit zum Reaktoreingang zurückgeführt und mit der umgepumpten Suspension innig vermischt. Der Wasserstoffverbrauch wird über die Frischwasserstoffleitung 4 kontinuierlich ergänzt. Über die Abgasleitung 10 kann eine bestimmte Menge Abgas gefahren werden, um eine Anreicherung von Inertgasen zu verhindern.

Nach beendeter Hydrierung wird die Suspension über die Entnahmeleitung 14 abgelassen.

Die hohe Raumzeitausbeute bei dieser Fahrweise wird dadurch erzielt, daß man die Suspension mit einer Querschnittsbelastung von 100 bis 500 $m^3/m^2.h$, vorzugsweise 150 bis 300 $m^3/m^2.h$, bezogen auf den freien Reaktorschnitt, umpumpt und über den Gasstrahlverdichter 5 den Wasserstoff optimal in der Suspension verteilt.

Innerhalb der Packungen wird bei dieser Fahrweise eine erhöhte Turbulenz in der begasten Suspension erzeugt. Dabei führen die Katalysatorpartikel gegenüber der Flüssigkeit eine verstärkte Relativbewegung aus, weil sie in den engen Öffnungen und Kanälen der Packungen eine Abbremsung gegenüber der sie umgebenden Flüssigkeit und den aufsteigenden Gasblasen erfahren.

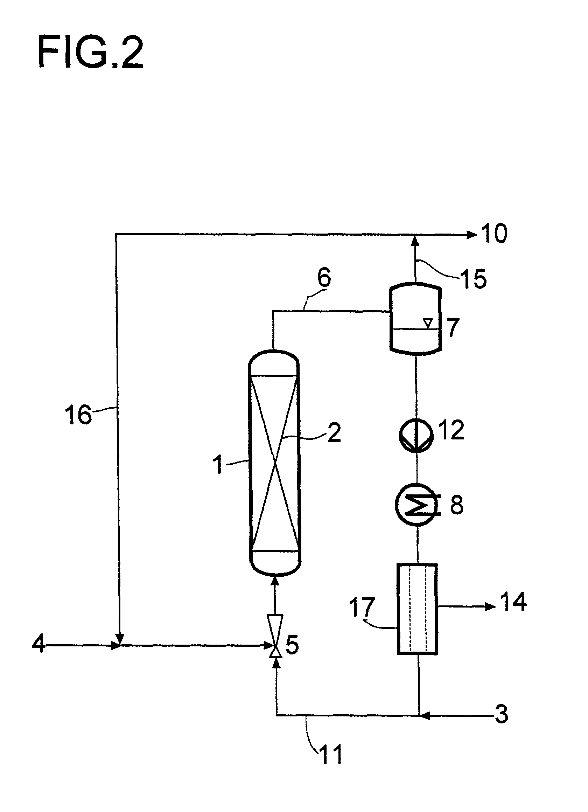

Fig. 2 stellt die besonders vorteilhafte kontinuierliche Fahrweise dar. Der Reaktor 1 ist mit einer Packung 2 gefüllt und mit einem Flüssigkeits- und Gaskreislauf versehen. Zuerst wird der gesamte Kreislauf mit Suspension, vorteilhaft bereits hydriertes Produkt und Suspensionskatalysator über die Zulaufleitung 3 gefüllt. Mittels der Kreislaufpumpe 12 wird die Suspension über den Vorheizer 8 und die Querstromfiltration 17 der Mischdüse 5 zugeführt. Die Mischdüse stellt einen Gasstrahlverdichter dar, der den Wasserstoff über die Kreisgasleitung 15 und 16 ansaugt und intensiv mit der Suspension vermischt. Ist der Kreislauf so in Betrieb genommen, wird über die Zuleitung 3 das zu hydrierende Edukt zugefahren. Der benötigte Wasserstoff wird mittels einer Druckhaltung über die $H_2$-Zuleitung 4 kontinuierlich zugeführt.

Suspension und Hydrierwasser werden im Reaktor 1 in den Öffnungen und Kanälen der Packungen innigst vermischt, so daß eine entsprechend gute Hydrierung erfolgt. Der Reaktoraustrag gelangt über die Leitung 6 in den Abscheider 7. Im Abscheider wird die Gasphase abgetrennt und über die Kreisgasleitung 15 und 16 zum Reaktoreingang zurückgeleitet. Über die Abgasleitung 10 kann eine bestimmte Menge Abgas ausgeschleust werden. Damit wird eine Anreicherung von Inertgasen im Wasserstoff vermieden.

Der supendierte Katalysator verbleibt im Reaktorsystem, indem er über den Querstromfilter 17 zurückgehalten wird. Das katalysatorfreie Produkt wird als Permeat über 14 abgeführt.

Für eine Produktionsanlage mit gepacktem Blasensäulenreaktor gemäß Fig. 2 betragen die Investitionskosten nur ca. 1/4 der Kosten, die für eine herkömmliche Rührkesselanlage gleicher Raumzeitausbeute aufzuwenden sind.

**Beispiel 1**

In den Reaktor einer Apparatur, die entsprechend der Figur 1 für diskontinuierliche Hydrierungen geeignet ist, wurden fünf Monolithe mit einem Durchmesser von 27 mm und einer Höhe von 5 cm, die aus $V_2A$-Gewebe, Werk-

stoff-Nr.1.4301, mit Kreuzkanalstruktur (Modul 1,0 mm) bestehen, eingefüllt. Das Metallgewebe in Leinenbindung besitzt eine Maschenweite von 0,18 mm und einen Drahtdurchmesser von 0,105 mm. Über einen Befülltrichter wurde die Apparatur mit 550 ml Citrallösung, bestehend aus 70 Gew.-% Citral, 27 Gew.-% Methanol und 3 Gew.-% Trimethylamin, sowie 5 g pulverförmigem Palladium/Kohle-Katalysator (5 Gew.-% Pd) beschickt. Letzterer besaß eine Korngrößenverteilung zwischen 0,001 und 0,2 mm mit einem 50% Wert von 0,012 mm, gemessen mit einem Cilas Laserspektrometer nach der Sedimentationsmethode gemäß DIN-Norm 66111. Nach dem Aufpressen von Wasserstoff auf 8 bar über die $H_2$-Zuführleitung 4 wurde die Kreislaufpumpe 12 in Betrieb genommen und eine Querschnittsbelastung von 200 $m^3/m^2$.h bezogen auf den freien Reaktorquerschnitt eingestellt. Die Düse des Gasstrahlverdichters saugte dann über die Kreisgasleitung 9 Wasserstoff an und begaste den Reaktor mit einer Gasgeschwindigkeit von 5,5 cm/s. Mittels des Vorheizers 13 wurde die Reaktoreintrittstemperatur auf 70°C gebracht. Während der Hydrierung wurden in bestimmten Zeitabständen Proben aus dem Kreislauf über das Ventil 14 entnommen und gaschromatisch analysiert.

Nach 2,75 h wurde ein Citralumsatz von 99,5% erreicht und eine Selektivität von 94%. Die Raumzeitausbeute, bezogen auf den Pd/C-Katalysator betrug: 22,4 kg Citral/$kg_{kat}$*h.

**Vergleichsbeispiel 1**

In einen 10 1-Rührkessel mit Begasungsrührer wurden 6,9 1 Citrallösung bestehend aus 70 Gew.-% Citral, 27 Gew.-% Methanol und 3 Gew.-% Trimethylamin, sowie 55 g pulverförmiger Palladium/Kohle-Katalysator (5 Gew.-% Pd) eingefüllt. Die Korngröße des Katalysators entsprach der in Beispiel 1.

Nach dem Aufpressen von Wasserstoff und Aufheizen des Kessels auf 70°C wurde unter Rühren mit 800 Umdrehungen pro Minute bei 8 bar $H_2$ hydriert. Durch Proben, die in bestimmten Zeitabständen genommen und gaschromatographisch untersucht wurden, konnte der Hydrierverlauf analysiert werden. Nach 19 Stunden betrug der Citral-Umsatz 99,7% bei einer Selektivität des Produkts von 92,9%. Die Raumzeitausbeute bezogen auf den pulverförmigen Pd/C-Katalysator betrug:

$$3{,}68\ kg_{Citral}/kg_{Kat.}{}^{*}h.$$

**Patentansprüche**

1. Verwendung eines gepackten Blasensäulenreaktors (2), dessen Zuführeinrichtung ein Gasstrahlverdichter (5) ist, mit Produktrückführung (6) und Wasserstoffkreisgas zur selektiven Flüssigphasenhydrierung von α,β-ungesättigten Carbonylverbindungen der allgemeinen Formel I,

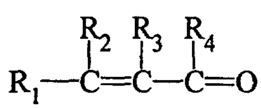

(I)

in der $R_1$ Wasserstoff oder ein organischer Rest, $R_2$, $R_3$ und $R_4$ unabhängig voneinander Wasserstoff oder eine $C_1$- bis $C_4$-Alkylgruppe ist, zu gesättigten Carbonylverbindungen der Formel II

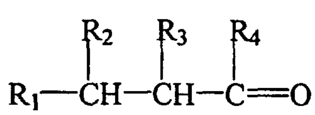

(II)

mit Wasserstoff in Gegenwart eines pulverförmigen Palladiumkatalysators und einer organischen Base.

2. Verwendung nach Anspruch 1, **dadurch gekennzeichnet, daß** die Packung des Blasensäulenreaktors Öffnungen und Kanäle aufweist, deren hydraulischer Durchmesser 0,5 bis 20 mm, vorzugsweise 1 bis 10 mm, besonders bevorzugt 1 bis 3 mm, beträgt.

3. Verwendung nach Anspruch 1 oder 2, **dadurch gekennzeichnet, daß** die Wandungen der Öffnungen oder Kanäle

der Packung des Blasensäulenreaktors Oberflächenrauhigkeiten im Bereich des 0,1- bis 10-fachen, vorzugsweise des 0,5- bis 5-fachen der mittleren Korngröße der pulverförmigen Katalysatorteilchen aufweisen.

**4.** Verwendung nach einem der Ansprüche 1 bis 3, **dadurch gekennzeichnet, daß** die Wandungen der Öffnungen oder Kanäle der Packung des Blasensäulen-reaktors metallisch sind und einen Mittenrauhwert $R_a$ nach DIN 4768/1 von 0,001 bis $0_2$01 mm aufweisen.

**5.** Verwendung nach einem der Ansprüche 1 bis 4, **dadurch gekennzeichnet, daß** die Packung des Blasensäulenreaktors aus metallischen Werkstoffen, Kunststoffen, Keramik und/oder anorganischen Fasern, insbesondere aus Kohlenstoff oder Asbestersatzstoffen hergestellt ist.

**6.** Verwendung nach Anspruch 5, **dadurch gekennzeichnet, daß** die Packung eine Folie, ein Gestrick oder Gewebe ist, wie es grundsätzlich aus der Destillations- oder Extraktionstechnik bekannt ist.

**7.** Verwendung nach einem der Ansprüche 1 bis 6, **dadurch gekennzeichnet, daß** der Palladiumkatalysator geträgert ist, wobei als Trägermaterial insbesondere Kohlenstoff, Zirkoniumdioxid oder Titandioxid, vorzugsweise mit einer mittleren Korngröße von 0,1 bis 300 µm, besonders bevorzugt mit einer mittleren Korngröße von 0,5 bis 100 µm, verwendet wird.

**8.** Verwendung nach Anspruch 7, **dadurch gekennzeichnet, daß** der Trägerkatalysator 0,01 bis 10 Gew.-% Palladium, vorzugsweise 0,2 bis 5 Gew.-% Palladium, besonders bevorzugt 0,5 bis 1 Gew.-% Palladium, enthält.

**9.** Verwendung nach einem der Ansprüche 1 bis 8, **dadurch gekennzeichnet, daß** die Flüssigphase mit einer Lehrrohrgeschwindigkeit von 100 bis 500 $m^3/m^2.h$, vorzugsweise 150 bis 300 $m^3/m^2.h$, umgepumpt wird.

**10.** Verwendung nach einem der Ansprüche 1 bis 8, **dadurch gekennzeichnet, daß** die Wasserstoffzufuhr mit einer Lehrrohrgeschwindigkeit von 0,5 bis 15 cm/s, vorzugsweise von 2,5 bis 10 cm/s, erfolgt.

**11.** Verwendung nach einem der Ansprüche 1 bis 10, **dadurch gekennzeichnet, daß** die Hydrierung bei Wasserstoffpartialdrücken von 1 bis 200 bar, vorzugsweise von 1 bis 100 bar, besonders bevorzugt von 1 bis 10 bar, durchgeführt wird.

**12.** Verwendung nach einem der Ansprüche 1 bis 11, **dadurch gekennzeichnet, daß** die Hydrierung bei Temperaturen von 25 bis 150°C, vorzugsweise von 50 bis 100°C, durchgeführt wird.

**13.** Verwendung nach einem der Ansprüche 1 bis 12, **dadurch gekennzeichnet, daß** es kontinuierlich durchgeführt wird.

**14.** Verwendung nach einem der Ansprüche 1 bis 13, **dadurch gekennzeichnet, daß** die Flüssigphase und der Wasserstoff im Gleichstrom gefördert werden, bevorzugt durch einen vertikal stehenden Blasensäulenreaktor von unten nach oben.

**15.** Verwendung nach einem der Ansprüche 1 bis 14, **dadurch gekennzeichnet, daß** die α,β-ungesättigte Carbonylverbindung der Formel (I) Citral ist.

## Claims

**1.** The use of a packed bubble column reactor (2) fed by a gas jet compressor (5) and equipped with product recycling (6) and circulating hydrogen gas for the selective liquid-phase hydrogenation of α,β-unsaturated carbonyl compounds of the formula (I)

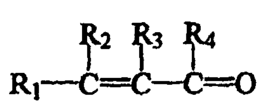

(I)

where $R_1$ is hydrogen or an organic radical, and $R_2$, $R_3$ and $R_4$, independently of one another, are hydrogen or a $C_1$- to $C_4$-alkyl group, to saturated carbonyl compounds of the formula (II)

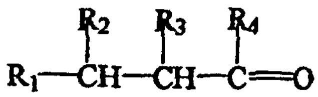

(II)

using hydrogen in the presence of a pulverulent palladium catalyst and in the presence of an organic base.

**2.** The use as claimed in claim 1, wherein the packing in the bubble column reactor has openings and channels whose hydraulic diameter is from 0.5 to 20 mm, preferably from 1 to 10 mm, particularly preferably from 1 to 3 mm.

**3.** The use as claimed in claim 1 or 2, wherein the walls of the openings or channels of the packing in the bubble column reactor have surface roughness values in the range from 0.1 to 10 times, preferably from 0.5 to 5 times, the mean particle size of the pulverulent catalyst particles.

**4.** The use as claimed in one of claims 1 to 3, wherein the walls of the openings or channels of the packing in the bubble column reactor are metallic and have a mean roughness $R_a$, measured in accordance with DIN 4768/1, of from 0.001 to 0.01 mm.

**5.** The use as claimed in one of claims 1 to 4, wherein the packing in the bubble column reactor is made of metallic materials, plastics, ceramics and/or inorganic fibers, in particular carbon or asbestos substitutes.

**6.** The use as claimed in claim 5, wherein the packing is a foil, gauze or mesh, as is known in principle from distillation or extraction technology.

**7.** The use as claimed in one of claims 1 to 6, wherein the palladium catalyst is supported, the support material used being, in particular, carbon, zirconium dioxide or titanium dioxide, preferably having a mean particle size of from 0.1 to 300 μm, particularly preferably having a mean particle size of from 0.5 to 100 μm.

**8.** The use as claimed in claim 7, wherein the supported catalyst contains from 0.01 to 10% by weight of palladium, preferably from 0.2 to 5% by weight of palladium, particularly preferably from 0.5 to 1% by weight of palladium.

**9.** The use as claimed in one of claims 1 to 8, wherein the liquid phase is circulated at a superficial velocity of from 100 to 500 $m^3/m^2.h$, preferably from 150 to 300 $m^3/m^2.h$.

**10.** The use as claimed in one of claims 1 to 8, wherein the hydrogen is fed in at a superficial velocity of from 0.5 to 15 cm/s, preferably from 2.5 to 10 cm/s.

**11.** The use as claimed in one of claims 1 to 10, wherein the hydrogenation is carried out at a hydrogen partial pressure of from 1 to 200 bar, preferably from 1 to 100 bar, particularly preferably from 1 to 10 bar.

**12.** The use as claimed in one of claims 1 to 11, wherein the hydrogenation is carried out at a temperature of from 25 to 150°C, preferably from 50 to 100°C.

**13.** The use as claimed in one of claims 1 to 12, which is carried out continuously.

**14.** The use as claimed in one of claims 1 to 13, wherein the liquid phase and the hydrogen are conveyed in cocurrent, preferably from bottom to top through a vertical bubble column reactor.

**15.** The use as claimed in one of claims 1 to 14, wherein the α,β-unsaturated carbonyl compound of the formula (I) is citral.

## Revendications

**1.** Utilisation d'un réacteur à colonne à bulles à garnissage (2) dont le dispositif d'alimentation consiste en un éjecteur à gaz (5), avec recyclage du produit (6) et hydrogène gazeux en circulation pour l'hydrogénation sélective en phase liquide de dérivés carbonylés alpha, bêta-insaturés de formule générale I

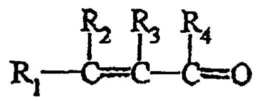 . (I)

dans laquelle $R_1$ représente l'hydrogène ou un radical organique, $R_2$, $R_3$ et $R_4$ représentent chacun, indépendamment les uns des autres, l'hydrogène ou un groupe alkyle en C1-C4, en dérivés carbonylés saturés de formule II

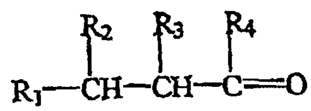 (II)

en présence d'un catalyseur au palladium en poudre et d'une base organique.

**2.** Utilisation selon la revendication 1, **caractérisée par le fait que** le garnissage du réacteur à colonne à bulles présente des orifices et canaux dont le diamètre hydraulique va de 0,5 à 20 mm, de préférence de 1 à 10 mm et plus spécialement de 1 à 3 mm.

**3.** Utilisation selon la revendication 1 ou 2, **caractérisée par le fait que** les parois des orifices ou canaux du garnissage du réacteur à colonne à bulles présentent des rugosités superficielles représentant de 0,1 à 10 fois, de préférence de 0,5 à 5 fois la dimension de grain moyenne des particules du catalyseur en poudre.

**4.** Utilisation selon l'une des revendications 1 à 3, **caractérisée par le fait que** les parois des orifices ou canaux du garnissage du réacteur à colonne à bulles sont métalliques et ont une valeur de rugosité moyenne $R_a$ selon DIN 4768/1 de 0,001 à 0,01 mm.

**5.** Utilisation selon l'une des revendications 1 à 4, **caractérisée par le fait que** le garnissage du réacteur à colonne à bulles consiste en matériaux métalliques, résines synthétiques, céramique ou fibres minérales, plus spécialement de carbone ou de produits de remplacement de l'amiante.

**6.** Utilisation selon la revendication 5, **caractérisée par le fait que** le garnissage consiste en une feuille, un tricot ou une toile du type connu en principe dans les techniques de distillation ou d'extraction.

**7.** Utilisation selon l'une des revendications 1 à 6, **caractérisée par le fait que** le catalyseur au palladium est un catalyseur sur support dont la matière de support consiste plus spécialement en carbone, dioxyde de zirconium ou dioxyde de titane, de préférence à une dimension de grain moyenne de 0,1 à 300 $\mu$m, plus spécialement à une dimension de grain moyenne de 0,5 à 100 $\mu$m.

**8.** Utilisation selon la revendication 7, **caractérisée par le fait que** le catalyseur sur support contient 0,01 à 10 % en poids de palladium, de préférence 0,2 à 5 % de palladium et plus spécialement 0,5 à 1 % en poids de palladium.

**9.** Utilisation selon l'une des revendications 1 à 8, **caractérisée par le fait que** la phase liquide est en circulation par pompe à une vitesse en tube calibré de 100 à 500 $m^3/m^2$.h, de préférence de 150 à 300 $m^3/m^2$.h.

**10.** Utilisation selon l'une des revendications 1 à 8, **caractérisée par le fait que** l'hydrogène est apporté à une vitesse en tube calibré de 0,5 à 15 cm/s, de préférence de 2,5 à 10 cm/s.

**11.** Utilisation selon l'une des revendication 1 à 10, **caractérisée par le fait que** l'hydrogénation est réalisée à des

pressions partielles d'hydrogène de 1 à 200 bar, de préférence de 1 à 100 bar et plus spécialement de 1 à 10 bar.

**12.** Utilisation selon l'une des revendications 1 à 11, **caractérisée par le fait que** l'hydrogénation est réalisée à des températures de 25 à 150° C, de préférence de 50 à 100° C.

**13.** Utilisation selon l'une des revendications 1 à 12, **caractérisée par le fait que** l'on opère en continu.

**14.** Utilisation selon l'une des revendications 1 à 13, **caractérisée par le fait que** la phase liquide et l'hydrogène sont transportés en courants parallèles, de préférence du bas vers le haut dans un réacteur à colonne à bulle verticale.

**15.** Utilisation selon l'une des revendications 1 à 14, **caractérisée par le fait que** le dérivé carbonylé alpha, bêta-insaturé de formule I et le citral.

FIG.1

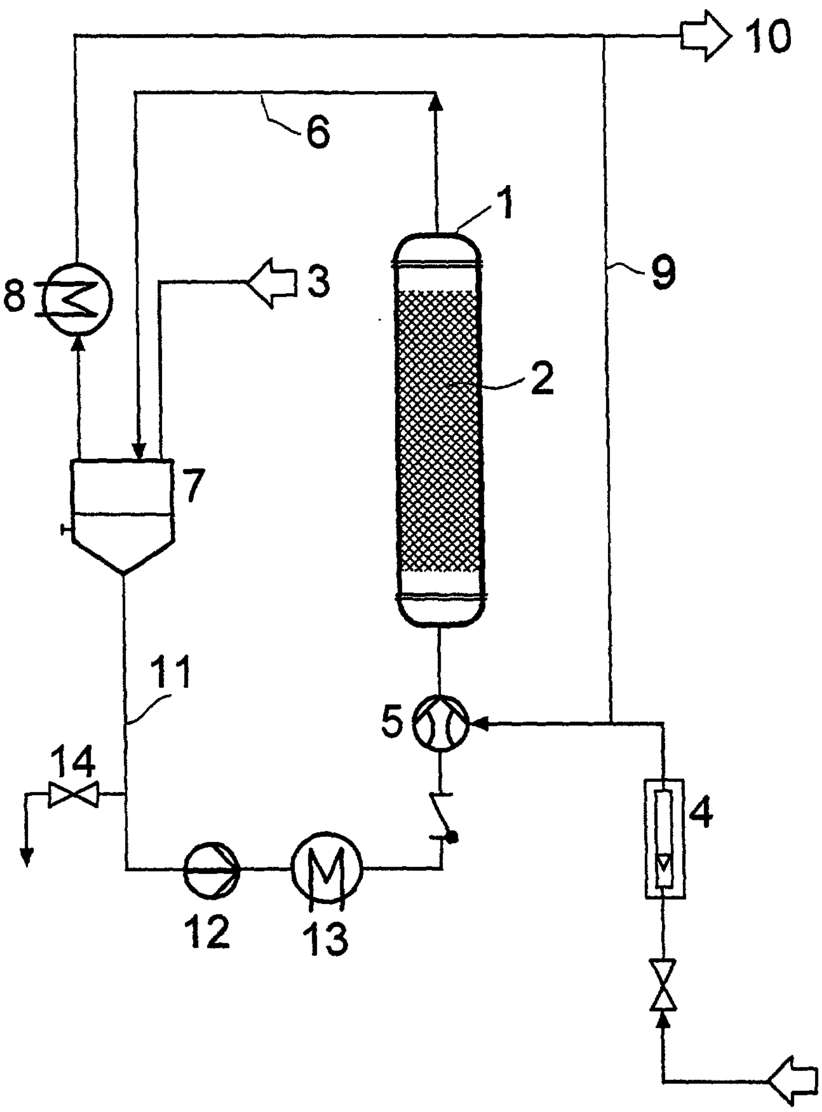

FIG.2